# EUROPÄISCHE PATENTANMELDUNG

(11) **EP 3 682 740 A1**
(43) Veröffentlichungstag der Anmeldung: **22.07.2020**
(21) Anmeldenummer: 19000029.9
(22) Anmeldetag: 16.01.2019
(51) Int. Cl.: A01N 61/00, A01N 65/00, A23B 7/16, A61K 8/92, A61K 8/06, C09D 193/00

(54) **ANTIMIKROBIELLE UND ANTIOXIDATIVE SOWIE MIKROBIZIDE ZUSAMMENSETZUNG UND DEREN VERWENDUNG**

(71) Anmelder: Popp, Alexander, 95213 Münchberg (DE)
(72) Erfinder: Popp, Alexander, 95213 Münchberg (DE)
(74) Vertreter: Helge, Reiner

(57) **Zusammenfassung**

Die Erfindung betrifft eine antimikrobielle und antioxidative sowie mikrobizide Zusammensetzung auf biologischer Basis als Emulsion, vorzugsweise einer Mikroemulsion, als Wachs in Natur - Öl Emulsion und deren Verwendung als Anstrich-, Beschichtungs- oder Konservierungsmittel.

Die antimikrobielle und antioxidative sowie mikrobiziden Zusammensetzung umfaßt als Wachs in Öl-Emulsion ein Mischungsverhältnis von mindestens 40 zu 60 und höchstens 2 zu 98, wobei natürlich vorkommende Wachse und Öle auf pflanzlicher Basis zum Einsatz kommen.

## Beschreibung

Die Erfindung betrifft eine antimikrobielle und antioxidative sowie mikrobizide Zusammensetzung auf biologischer Basis als Wachs in Öl-Emulsion, vorzugsweise als Mikroemulsion, wobei natürlich vorkommende Wachse und Öle auf pflanzlicher Basis zum Einsatz kommen, und deren Verwendung als Anstrich-, Beschichtungs- oder Konservierungsmittel.

Anstrich- und Beschichtungsmittel werden zum Zwecke des Oberflächenschutzes auf Oberflächen vorzugsweise mittels Streichen, Rollen oder Sprühen aufgetragen welche einen physikalisch trocknenden Anstrich ergeben, welcher zur Verwendung als Beschichtung geeignet ist. Zum Teil werden diese Anstrichmittel technisch aufwendig aus synthetischen Bindemitteln, welche der Petrochemie entstammen, aus Pigmenten und Hilfsmitteln hergestellt. Teilweise werden Anstrichmittel auch mit Bindemitteln natürlichen Ursprung hergestellt. Als natürliche Öle werden z.B. Leinöl oder Leinöl-Standöl verwendet unter Zusatz von Trockenstoffen, meist Octoaten und Linoleaten mit Kobalt, Mangan, Blei, Zirkonium oder anderen Metallen als Metallkomponente um die Trockenzeiten zu verkürzen. Durch diese Trockenstoffe werden somit zum Teil gesundheitsschädliche Metallionen sowie Lösemittel der Petrochemie in das Anstrichmittel eingeführt.

Als Konservierungsmittel werden antimikrobielle Biozide zur Tötung oder Wachstumshemmung von Mikroorganismen eingesetzt. Sie kommen zum Einsatz, wenn physikalische Methoden alleine nicht ausreichen. Das Spektrum möglicher Verbindungen hat sich seit längerem nicht geändert, da es schwierig ist, billige Stoffe mit breiter Wirkung und geringer Toxizität für Säugetiere zu finden. Bei der Verwendung von schwachen Säuren ist deren pK-Wert und ein saurer pH-Wert im Lebensmittel notwendig, da nur undissoziierte Moleküle durch die Zellmembran in das innere von Mikroorganismen eindringen können.

Man unterscheidet Lebensmittelzusatzstoffe, die den Verderb von Lebensmitteln z. B. durch Bakterien, Hefe- und Schimmelpilze verhindern sollen. Sie spielen eine wichtige Rolle in der Lebensmittelkonservierung und verhindern gefährliche Krankheiten wie Botulismus und Listeriose.

Bei zahlreichen Kosmetika ist eine Konservierung notwendig. Oft werden Kosmetik-Inhaltsstoffe, wie beispielsweise Parabene, Benzoesäure oder Methylisothiazolinon als Konservierungsmittel eingesetzt (werden). Es dürfen aber nur die Konservierungsmittel verwendet werden, die nach der Verordnung (EG) Nr. 1223/2009 über kosmetische Mittel zulässig sind.

Biozide kommen in unterschiedlichsten Anwendungsgebieten zum Einsatz, um das Wachstum von Mikroorganismen zu kontrollieren oder zu verhindern, und damit die Konservierung von Produkten und Materialien zu gewährleisten. Sie finden Anwendung in der Baustoff-, Textil-, Leder-, Papier- und Lebensmittelindustrie, aber auch in der Kosmetik.

Bekannt ist aus der DE 20 2006 013 769 U1 ein antimikrobielles Mittel zur natürlichen Behandlung von Holzoberflächen unter Einsatz von möglichst homogen verteiltem nanopartikulärem Silber wobei es sich bei dem Holzschutzmittel um Schellack oder natürliches Wachs oder natürliches Öl handelt.

Bekannt ist aus der DE 10 2006 019 818 A1 eine wässrige Wasserdispersion zur Imprägnierung von Lignocellulose-Materialien, wobei die wässrigen Dispersionen der Wachse oder wachsartigen Polymere dadurch gekennzeichnet sind, dass der Wachsbestandteil einen Schmelzpunkt oberhalb 75 °C, vorzugsweise oberhalb 80 °C, insbesondere oberhalb 90 °C aufweisen und die dispergieren Wachspartikel eine mittlere Teilchengröße unterhalb 500 nm aufweisen.

Aus der DE 000009412715U1 ist eine abbaubare Wachskomposition und daraus hergestellte Wachsemulsion, die für alle bekannten Wachsanwendungen einsetzbar sind, bekannt. Die Erfindung betrifft auch wässrige Emulsionen auf Basis der erfindungsgemäßen
insitu-Wachskompositionen wobei die wässrigen Emulsionen 10-40 Gewichtsanteile der erfindungsgemäßen in situ Wachskomposition, 90-60 Gewichtsanteile Wasser, vorzugsweise entsalztes Wasser, 0-15 Gewichtsanteile eines Anverseifungsmittels enthalten.

Bekannt ist aus der DE 198 41 271 A1 ein Holzschutzmittel zum Auftragen auf zu behandelnde Holzoberflächen, bestehend aus einem Naturöl oder Bioöl oder aus Mischungen von Naturölen oder Bioölen, vorzugsweise Olivenöl, Öl der Ölpalme, Avocadoöl, Rapsöl, Lein- oder Flachsöl, Sojaöl, Baumwollöl, Erdnussöl, Sonnenblumenöl, Kürbisöl, Rizinusöl, Mohnöl, Sesamöl, Kokosnussöl, Kakao-und Mandelöl, Maisöl in beliebigen Verhältnissen und zur Farbgestaltung unter Zugabe pflanzlicher und/oder chemischer Farbstoffe gemischt.

Bekannt ist aus der DE 198 47 964 A1 ein ökologisch unbedenkliches, pflegendes Holzschutz-und Holzkonservierungsmittel mit Langzeitschutz gegen Tropenholzschädlinge, insbesondere Insekten und Mikroorganismen, wobei als Trägermaterial und Bindemittel ein Fettsäurealkülester-Gemisch (Rapsölfettsäuremethylester-Gemisch) verwendet wird.

Bekannt ist aus der DE 10 2005 002 096 B3 ein Imprägniermittel zur Imprägnierung von fertig ausgetrocknetem und profiliertem Holz, bestehend aus einem Stoffgemisch von wenigstens 2 Komponenten aus den Stoffgruppen, Paraffin, Paraffinwachse, pflanzliche Wachse, pflanzliche Öle und/oder Silikonwachse, bei der sich 2 Komponenten hinsichtlich ihrer Mobilität unterscheiden, wobei die wenigstens eine mobilere Komponente des Imprägniermittels unter den Umgebungsbedingungen migrationsfähig ist

Bekannt ist aus der EP 0 798 349 A2 ein Mittel zum Aufbringen eines Schutzüberzugs auf harten Oberflächen, um sie gegen Witterungseinflüsse und mechanische Beanspruchung sowie gegen Einwirkung von Chemikalien zu schützen, a) bestehend aus einer Wirkstoffkomponente, bestehend aus mindestens einem natürlichen und/oder
synthetischen Wachs, mindestens einem Polymer oder Vorläufer davon und gegebenenfalls für pyrogener Kieselsäure, b) einer flüssigen Trägerkomponente, bestehend aus mindestens einer organischen Flüssigkeit und/oder Wasser, und c) gegebenenfalls einem oder mehreren üblichen Zusätzen aus der Gruppe der Netzmittel, Verlaufmittel, Dispergiermittel, Emulgiermittel, Stabilisatoren, Schutzkolloide, Füllstoffe, Riechstoffe, Konservierungsmittel, Hautschutzmitteln, Markierungsmittel, Pigmente, Farbstoffe und/oder Farbtönungsmittel.

Bekannt ist aus der DE 4303415A1 ein Holzkonservierungsmittel, vorzugsweise Vollschutzanstrichmittel ohne Pigmente, Farbstoffe, Korrosionsschutzmittel oder andere Zusatzstoffe, vorzugsweise Topfkonservierungsmittel, Antiabsatzmittel, Wachse oder Verbindungen mit wachsähnlichen Eigenschaften.

Bekannt ist aus der DE 39 42 136 A1 ein Hartwachs enthaltendes Imprägniermittel zum Schutz von Holz-, Kork-, Kultur-, Naturstein-und lackierten Metalloberflächen, enthaltend Wachs, Öl, Harz und übliche Zusätze.

Bekannt ist aus der DE 43 03 415 A1 eine Emulsion enthaltend Chitosan, Wasser, Säure, Öl und/oder Wachs, die als gebrauchsfertiger Stammansatz herstellbar ist und so weiterverwendet werden kann.

Bekannt ist aus der DE 10 2007 009 450 A1 eine antimikrobielle Zusammensetzung, enthaltend Silber in Form von metallischem Silber oder als Silberverbindung/en und mindestens einer weiteren bioziden Wirksubstanz, ausgewählt aus der Substanzklasse der Isothiazoline und deren Verwendung als Konservierungs- und Desinfektionsmittel.

Ausgehend vom vorbeschriebenen Stand der Technik besteht die Aufgabe der Erfindung darin, eine antimikrobielle und antioxidative sowie mikrobizide Zusammensetzung auf biologischer Basis als Emulsion, vorzugsweise einer Mikroemulsion, als Wachs in Öl-Emulsion und deren Verwendung als Anstrich-, Beschichtungs- oder Konservierungsmittel bereitzustellen, welche atmungsaktiv, zugleich UV- stabil bzw. UV- und alterungsbeständig sowie see- und salzwasserfest ist, bei einem Auftrag auf einer Holzoberfläche die natürliche Holzfarbe und Maserung vollständig und ohne Beeinträchtigung erhält, bei Auftrag auf Oberflächen von Textilien eine Speichel - und Schweißechtheit gewährleistet sowie dauerelastische Eigenschaften aufweist und darüber hinaus insbesondere frei von Lösungen aus Terpentinen, Paraffinen, Silikon-, Teflon- und Mineralölen, Pigmenten und jeglichen Konservierungsstoffen und Zusatzstoffen wie beispielsweise Primern oder Sikkativen ist, gleichwohl eine wirksame antimikrobielle und antioxidative sowie mikrobizide Wirkung, vorzugsweise wundheilende und vernarbende Wirkung sowie eine bienen- und nutzinsektenfreundliche Wirkung bei 100 % pflanzlicher Herkunft aufweist.

Eine weitere Aufgabe der Erfindung besteht auch darin, ein Anstrich- bzw. Beschichtungsmittel mit der erfindungsgemäßen Zusammensetzung bereitzustellen, welches universell einsetzbar, kalt auftragbar und auf allen Oberflächen, auch auf grobporigen Innen- und Außenputzen sowie auf Fassadenanstrichen auf Mauerwerken, auf Dachfirst- und sonstigen Dacheindeckungen wie Firstblechen, selbstreinigenden Dächern oder Flächen gut haftbar ist.

Ferner besteht eine weitere Aufgabe darin, die erfindungsgemäße Zusammensetzung als Konservierungsmittel und Glanzmittel für Obst und Gemüse, im speziellen als Konservierungsmittel für Trockenfrüchte bzw. Dörrobst sowie als Überzugs- und Glanzmittel bei z.B. Nahrungsergänzungsmitteln, sowie als aluminium-, benzoesäure-, methylisothiazolinon-, PEG- und parabenfreies Konservierungsmittel zur Anwendung in der Natur- und Bio-Kosmetik bereitzustellen.

Eine oder mehrere dieser Aufgaben werden erfindungsgemäß durch eine antimikrobielle und antioxidative, sowie auch mikrobizid keimtötende Zusammensetzung auf biologischer Basis als Wachs in Öl-Emulsion, vorzugsweise als Mikroemulsion, wobei natürlich vorkommende Wachse und Öle auf pflanzlicher Basis zum Einsatz kommen, mit den Merkmalen des kennzeichnenden Teils des Hauptanspruchs 1 gelöst. Weitere bevorzugte erfindungsgemäße Ausgestaltungen sind den Unteransprüchen zu entnehmen.

Die Erfindung betrifft eine Wachs in Öl Emulsion in einem Verhältnis von Öl zu Wachs von mindestens 60 zu 40, höchstens von 95 zu 5 Gewichtsteilen, bevorzugt jedoch ein Verhältnis von Öl zu Wachs von 75 zu 25.

Eine weitere bevorzugte Ausführung der Erfindung besteht darin, für die Emulsion das Verhältnis von Öl zu Wachs von 80 zu 20 bis 95 zu 5 zu verwenden, insbesondere bevorzugt wird ein Verhältnis von Öl zu Wachs von 90 zu 10. Bei weiteren spezifischen Anwendungen wird ein Verhältnis von Öl zu Wachs von 98 zu 2 verwendet. Die Zusammensetzungen können in jedem Falle leicht variieren.

Für sämtliche Verhältnisse von Öl zu Wachs der Emulsion sind vorzugsweise biologisch abbaubare natürliche Öle und Wachse ausschließlich aus nachwachsenden Rohstoffen zu verwenden. Zu verwenden sind kaltgepresste pflanzliche Öle, frei von jeglichen chemischen Pflanzenschutzmitteln und Pestiziden. Bevorzugt werden Öle aus mediterranen Ölfruchten, die teilweise winterhart kultiviert werden. Insbesondere bevorzugt wird das Öl aus Ölfrüchten, vorzugsweise dessen der Olive, in bestimmten Fällen auch ein Lampanöl. Darüber hinaus kommt ein Hanfkernöl zur Anwendung, dass sehr gut in die Haut einzieht. Es wird im speziellen auch ein CBD - Öl der Hanfblüte, dass in Alkohol destilliert und extrahiert wird, als Additiv verwendet. Das kaltgepresste Öl der Samen ist vom Fettsäurenmuster enorm wertvoll, da es über einen hohen Anteil an Gamma-Linolen-Säuren (GLA) verfügt.

Zur Anwendung kommen natürliche Wachse, wie Bienen- oder Carnaubawachse oder Wollwachse (Lanolin), insbesondere das kalt abgeschabte Wachs der Apfelschalenwachsschicht als wichtigster Bestandteil. Diese wird u.a. aus der Anzucht eigens veredelter hochstämmigen Sorten, die ihren Ursprung aus nachhaltigen Streuobstwiesen finden. Diese Streuobstwiesen erfüllen gleichzeitig die Aufgabe eines zukünftigen Genpools und fungieren als natürliche Barrikaden gegen Bodenerosion und dienen der Beschattung in Zeiten zunehmender Trockenheit. Sie werden nicht in Plantagen und Monokulturen kultiviert oder gentechnisch verändert gewonnen. Der Einsatz jeglicher Pflanzenschutzmittel ist hierbei gänzlich untersagt. Unter diesen Bäumen soll als Zusatzeffekt darüber hinaus eine ökologische Land- und Viehwirtschaft betrieben werden, die keine weitere Flächenverknappung hervorruft. Es handelt sich um spezielle Sorten, die keine Mineraldüngergaben verabreicht bekommen.

Ein Beschichtungs- und Pflegemittel , insbesondere eine Spinnlösung für natürliche Regeneratfasern wie Cellulose- bzw. Viskosefasern. Darüber hinaus fungieren die Wirkstoffe als UV - Schutz bei Aramiden bzw. aromatischen Polyamiden, die bei Sonneneinstrahlung an Bruchlast verlieren, sowie auch als Beschichtung gegen Feuchtigkeitseintritt u.a. der Luftfeuchtigkeitsaufnahme aus der Umgebungsluft, da hierbei ohne Schutz eine Längenausdehnung in Faserrichtung erfolgt bei der eine unerwünschte Veränderung der Webstruktur auftritt.

Für Oberflächen von Textilien, wie Plüsche und Velours, die bei Kinderspielsachen wie z.B. Kuscheltieren Anwendung finden und eine Schweiß- und Speichelechtheit aufweisen müssen, atmungsaktive, wind-, schmutz- und wetterabweisende biologische Imprägnierung für Funktions-, Arbeits- und Winterbekleidung, Anwendung auf nachhaltiger Bekleidung, Ausweichoption für bedenkliche Imprägniermittel mit Inhaltsstoffen wie Nanopartikel. Hydrophob- und Oleophobausrüstung von Textilien, antimikrobielle und mikrobizide Behandlung, Beschichtung von Wetterschutzabdeckungen für Freiflächen wie z.B. Markisenstoffen, Zelten und Campingausrüstungen. Eine Behandlung von Textilien technischer Art und Vliessstoffen, u.a. auf dem Gebiet der Filtration, als Avivage für z.B. Pollenfilter oder Filtereinsätzen in Wohnraumlüftungsanlagen, die für eine schadstofffreie Raumluft sorgen und eine keim-, schimmel- und pilzhemmende Wirkung besitzen und keinerlei Geruchsbelästigung in Wohnräumen aufweisen dürfen, machen sich die genannten positiven Eigenschaften deutlich bemerkbar. Diese Beschichtung lässt einen Einsatz in biologisch abbaubaren Reinigungs- und Desinfektionstüchern zu.

Die Verwendung der erfindungsgemäßen antimikrobiellen und antioxidativen Zusammensetzung kann als Anstrichmittel, Beschichtungs- und Konservierungsmittel erfolgen. Insbesondere kann die Zusammensetzung als Anstrichmittel für Holz, Natur- oder Kunststein, aber auch aufgrund der See- und Salzwasserbeständigkeit als Anstrichmittel für Schiffs- und Bootsrümpfe als Antifouling verwendet werden, da ein Ansiedeln von Algen und sonstigen Organismen verhindert wird. Im Weiteren kann die Zusammensetzung auch als Imprägniermittel verwendet werden.

Die Verwendung der erfindungsgemäßen antimikrobiellen und antioxidativen Zusammensetzung kann als Konservierungs- und Glanzmittel bei Schalenobst, z. B. bei Citrusfrüchten - wie Orangen und Zitronen, die bedenkliche fungizidhaltige Pflanzenschutzmittel, wie z. B. Imazalil, Pyrimethanil sowie Thiabendazol oder Phenylphenole, die als krebserregend gelten, ersetzen kann, da dass Wachs-/Ölgemisch in Wasser nicht löslich bleibt und auf Oberflächen einen dauerhaften Glanzfilm hinterläßt. Die bedenklichen Inhaltsstoffe der am Markt eingesetzten Glanz- und Konservierungsstoffe landen in großen Mengen in der "Biotonne" und gelangen über die Verkompostierung unzersetzt wiederrum in den Nahrungskreislauf.

Die Atmungsaktivität und die dauerelastichen Haftungseigenschaften der erfindungsgemäßen Zusammensetzung fungieren als natürliches Pflaster.

Die erfindungsgemäße Zusammensetzung eignet sich in dieser Form auch als Anstrichmittel für Bienenbehausungen , um die direkte Verbindung der Propolisschicht zur Umwelt hin nicht zu beeinträchtigen.

Es wird in der reinen Wachsform weiterhin eine Konservierung von alten und wertvollen Gemälden gewährleistet, um ein ausbleichen aufzuhalten.

Besonders häufig finden sich PEGs in Tensiden von Shampoos und Duschgelen. Im Besonderen können die Inhaltsstoffe für Gesichts- bzw. Tuchmasken, in der Haarpflege zur Haarspitzenversiegelung und als Sonnenschutzcreme verwendet werden. Die Mischung verleiht Glanz und Geschmeidigkeit, ohne einen fettigen Rückstand zu hinterlassen.

Die natürlichen Inhaltsstoffe können dazu beitragen, die Feuchtigkeit der Haut zu erhalten und schützende Zellschichten zu stärken, sowie beschädigte Zellschichten zu regenerieren. Diese bilden wichtige Barrieren und schützen die Haut vor Rissen, Irritation und vor dem Austrocknen sowie einem vorzeitigen Altern. Die Emulsion verhindert die Aufnahme und das Eindringen von Schadstoffen sowie weiteren negativen Umwelteinflüssen. Der eingeschränkte Feuchtigkeitsverlust der Haut kann durch Auffüllen von Lücken und Unebenheiten durch die Wachsanteile aufrechterhalten werden.

Als parfümfreie und geruchsneutrale Zusätze wirken die Inhaltsstoffe konturenfestigend und bewirken eine natürliche und effiziente Anwendung auf Anti Aging - Gesichts- und Körpercremes sowie einer Anti - Falten - Pflege für Tag- und Nachtanwendungen bei anspruchsvollen und sensiblen Hauttypen, die darüber hinaus einer frühzeitigen Hautalterung entgegenwirken. Faltentiefen können um 25% minimiert werden. Die regenerierenden Eigenschaften hinterlassen ein sehr und beruhigendes Gefühl auf der Haut und ziehen sehr gut ein. Es wurde darüber hinaus eine leichtere Schmutzentfernung festgestellt, diese ermöglicht einen Einsatz als transparenten Hautschutz. Die Emulsion kann als Konsistenzgeber oder Perlglanz in verschiedenen Cremes, Deos oder Zahnpasta zum Einsatz kommen. Es sind keinerlei Mikroplastiken und Acrylate enthalten. Es ist eine klare bis honiggelbe Mischung, die sehr haltbar und nahezu geruchslos ist.

Die Erfindung wird anhand des folgenden Ausführungsbeispiels näher erläutert:

Die Emulsion ist mit Inhaltsstoffen und einer Zusammensetzung pro 100 g der Emulsion wie folgt gekennzeichnet: Hauptbestandteil: kaltgepresstes Öl aus Ölfrucht, 90,63 g, wobei das Öl einen Säuregehalt (Acidity) bzw. einen Anteil der freien Fettsäuren bis maximal 0,8 % aufweist und bei einem Fettgehalt von 90,63 g/100 g gesättigte Fettsäuren 14,6 g davon 70 g einfach ungesättigte Fettsäuren und davon 6 g mehrfach ungesättigte Fettsäuren enthält.

Das Oel weist einen Delta K-Wert von maximal 0,01, einen K270-Wert von 0,15 bis maximal 0,22 und einen K232-Wert von maximal 2,5 auf. Der Delta K-Wert kennzeichnet die Reinheit des Öls, der K232-Wert die bei der Oxidation entstehenden konjugierten Diene und der K270-Wert die bei fortschreitender Oxidation entstehenden konjugierten Triene. Allgemein ausgeführt geben die Werte die Frische und die Reinheit des Öls an. Des Weiteren sollen die Öle keine Salze, Proteine, Ballaststoffe, Zucker und Kohlenhydrate enthalten und nicht den Erdölen und seinen Derivaten zuord (en) bar sein.

Als Wachsbestandteil wird dem Öl Apfelschalenwachs zugesetzt, wobei 9,37 g dieses 48 % aliphatische, nicht den Erdölen und seinen Derivaten zugeordnete Kohlenwasserstoffe beinhaltet.

Im flüssigen Zustand des Apfelschalenwachses sind im Einzelnen enthalten Pentadecan, Hexadecan, Octacosan, Tetracosan, Heptadecan, Nonadecan, Stearylalkohol, Eicosan, 19 % Ester, 25% freie Fettsäuren, 2 % Alkohole (Stearyalkohol, Cetylalkohol in Spuren) und 6 % Aromen.

Durch die Verwendung von natürlichen, kaltgepressten und ohne Lösungsmittel extrahierten Ölen u.a. einer winterhart kultivierten speziellen mediterranen Ölfrucht (Olivenverwandtschaft) ist insbesondere gegenüber sonstigen Ölen wie Leinöl, Rapsöl, Walnussöl, Sonnenblumenöl, Sesamöl und Erdnussöl eine besonders vorteilhafte fungizide, antibakterielle, mikrobizide, entzündungshemmende und der Wundheilung sowie einer Vernarbung fördernden Wirkung festzustellen.

Aufgrund des geringen Schmelzbereiches von ca. 40 °C und der Vermischung bei max. 60 °C bleibt die Emulsion kalt aufbringbar, hat eine dem Lotuseffekt gleiche selbstreinigende und schmutzabweisende Wirkung und haftet auf allen Oberflächen, insbesondere zur Verwendung als Wetterschutzöl auf Holzoberflächen und zur Imprägnierung von Lignocellulosematerialien sowie einer Beschichtung und Versiegelung auf Holzböden und Terassendielen nach innen hin, die einer vorzeitigen Verwitterung und Alterung aufgrund direkter Sonneneinstrahlung und der Witterung ausgesetzt, vorbeugend angewandt werden können.

Die Verwendung als antibakterielle Behandlung von grobporigen Innen-und Außenputzen, Beton, gefüllten Untergründen, Glasfasergeweben und Glasvlies sowie Fassadenanstrichen auf Mauerwerken aber auch auf Blei- und Kupferbeschichtungen bei Dachfirst- und sonstigen Dacheindeckungen wie Firstblechen sowie selbstreinigenden Dächern, zeigt sich als äußerst wirksam. Das Pflegeöl wird auch zur Sättigung von strapazierten und verblassten Holzoberflächen, wie auf z.B. Fenster- und Türrahmen sowie -flügeln verwendet.

Durch die Verwendung von natürlichen Ölen besteht der Vorteil des Anstrichmittels darin, nach dem Auftrag als trockener Anstrich gleichwohl atmungsaktiv und einem nach außen hin atmosphärischen Austausch offen und widerstandsfähig gegen jegliche Freibewitterungsfaktoren, wie Temperatur, Luftsauerstoffgehalt, Ozon, relative Luftfeuchtigkeit, UV-Einstrahlung, Umweltverschmutzung und Abgasen (Kohlenmonoxid, Kohlendioxid, Schwefeldioxid, Stickoxide) und Salzwasser zu sein.

Die Überlegenheit der erfindungsgemäßen Zusammensetzung als Anstrichmittels besteht darin, dass in der Grundausführung weder künstliche noch natürliche Farbstoffe zugesetzt sind und bei einem Auftrag des Anstrichmittels auf einer Holzoberfläche die natürliche Holzfarbe und Maserung vollständig und ohne Geruchs- und Farbechtheitsbeeinträchtigung erhalten bleibt, gleichwohl das Material Speichel-und Schweißecht ist (speichel- und schweißecht bleibt).

Eine Anwendung auf Anstrichen aller Art, wie auch auf Lehmfarbanstrichen, ermöglicht eine qualitative Farbauffrischung und Sättigung sowie eine bessere Atmungsaktivität und sorgt gleichzeitig für einen Erhalt der Raumluftreinheit. Auf einen Einsatz von Schwermetallen und bedenklichen Titandioxiden kann verzichtet werden. Darüber hinaus können je nach Einsatzbereich, ätherische Öle oder natürliche Farbzusätze wie Anthocyane aus z.B. Rote Bete, Holunder oder Heidelbeere gewonnen, sowie spezielle natürliche Farbpigmente zugesetzt werden. Eine Ausmischung nach RAL bzw. NCS bleibt dabei uneingeschränkt.

Durch die Verwendung von natürlichen Ölen und Wachsen bei 100 % pflanzlicher Herkunft besteht der Vorteil der Emulsion weiter darin, gänzlich frei von Paraffin, Silikon-, Teflon- und Mineralölen, Pigmenten und jeglichen Konservierungsstoffen, auch synthetischer Art und Zusatzstoffen, wie z.B. Primern oder Sikkativen sowie Styrolen zu sein. Damit ist die Emulsion biologisch abbaubar und ohne jegliche gesundheitsschädliche Zusatzstoffe, Lösemittel oder Bindemittel der Petrochemie, insbesondere ohne gesundheitsschädliche Metallionen auch unter gesundheitsspezifischen Aspekten universell einsetzbar und verfügt über eine lange Haltbarkeit. Die Mikroemulsion bleibt dabei ohne Beeinträchtigung streich-, sprüh-, farbroll- sowie tränk- und tauchbar und behält ihr gutes Dispergiervermögen.

Die Verwendung von kalt abgeschabtem Wachs der Apfelschalenwachsschicht eines speziellen Winterapfels stellte sich als besonders vorteilhaft heraus. So konnte festgestellt werden dass es sich bei der Fruchtschale der Apfelschale um eine dicke und transparente Schalenwachsschicht handelt welche kalt geschabt und extrahiert die für die erfindungsgemäße Emulsion vorteilhaften Eigenschaften aufweist. Die Fruchthaut - Epidermis mit aufliegender Kutikula und deren Verschluss mit Wachs, fixiert die durch dessen Oberflächenexpansion erzeugte Spannung im Cutin - Gerüst auf den Wert 4,5. Durch die Aufzucht verschiedener Sorten zeigt sich bei der Ernte, das ein Zeitraum über mehrere Monate durch das Abreifen der verschiedenen Obstsorten abgedeckt werden kann.

Im Holzbau kann die Bekämpfung von Trockenholzschädlingen rückstandsfrei angewandt werden. Am Ende der Nutzungs- bzw. Lebensdauer bei z.B. Holzhäusern muß keine Problemmüllentsorgung für Fassadenteile etc. erfolgen. Es entsteht keinerlei Sondermüll, wie es u.a. bei formaldehyd- oder acrylharzbindenden Anstrichen der Fall ist. Eine Verschmutzung vom Grundwasser wird ausgeschlossen, da der Anstrich gänzlich biologisch abbaubar ist und jeglichen Anforderungen des Gewässerschutzes erfüllt. Es werden keine gesundheitsschädlichen Schleifstäube frei, wie es bei einem Überschleifen von am Markt erhältlichen Holzschutzlasuren der Fall ist. Der Einsatz lässt eine Zulassung in allen Gesichtspunkten der Baubiologie zu.

Die Verwendung als polierfähiges Naturwachs in Verbindung mit dem Zusatz von Baumharzen für antistatische Hartwachsanwendungen oder Fahrzeugwäschen und Lackaufbereitungen, insbesondere durch dessen sehr geringen Säuregehaltes, lässt einen Einsatz als Mikrowachs zu und wird zur Oberflächenversiegelung von Holz, Lignocellulose - Materialien, Leder, Glas, Metall, Marmor und Steingut, Kunststoff, Keramik, Elfenbein, als Ersatz für Bohnerwachs und bei Skibelagsaufbereitungen wie auch Steigfellen eingesetzt. In Verbindung mit sehr feinen Tonerden, die frei von jeglichen Mikroplastiken und Aluminium sind, kann darüber hinaus eine gründliche und schonende Reinigung von Oberflächen aus Glas, Kunststoff oder Metall, aber auch für lackierte Metalloberflächen erfolgen, in einem hautverträglichen und handschonenden Milieu mit einem hautfreundlichem physiologischen pH - Wert.

## Patentansprüche

1. Antimikrobielle und antioxidative sowie mikrobiziden Zusammensetzung
**dadurch gekennzeichnet, dass**
die Zusammensetzung als Emulsion auf biologischer Basis in Form einer Wachs in Öl-Emulsion mit einem Mischungsverhältnis von mindestens 40 zu 60 und höchstens 2 zu 98 ausgebildet ist, wobei natürlich vorkommende Wachse und Öle auf pflanzlicher Basis zum Einsatz kommen.

2. Antimikrobielle und antioxidative sowie mikrobiziden Zusammensetzung nach Anspruch 1,
**dadurch gekennzeichnet, dass**
die Zusammensetzung bevorzugt ein Mischungsverhältnis von 25 zu 75 aufweist.

3. Antimikrobielle und antioxidative sowie mikrobizide Zusammensetzung nach Anspruch 1,
**dadurch gekennzeichnet, dass**
die Zusammensetzung bevorzugt ein Mischungsverhältnis von 10 zu 90 aufweist.

4. Antimikrobielle und antioxidative sowie mikrobizide Zusammensetzung nach Anspruch 1,
**dadurch gekennzeichnet, dass**
die Zusammensetzung bevorzugt ein Mischungsverhältnis von 2 zu 98 aufweist.

5. Antimikrobielle und antioxidative sowie mikrobizide Zusammensetzung nach Anspruch 1 bis 4,
**dadurch gekennzeichnet, dass**
als Öl ein kaltgepresstes pflanzliches Öl verwendet wird.

6. Antimikrobielle und antioxidative sowie mikrobizide Zusammensetzung nach Anspruch 1 bis 5,
**dadurch gekennzeichnet, dass**
als Öl ein Olivenöl, ein Hanfkernöl bzw. ein Rapsöl oder ein Gemisch daraus verwendet wird.

7. Antimikrobielle und antioxidative sowie mikrobizide Zusammensetzung nach Anspruch 1 bis 6,
**dadurch gekennzeichnet, dass**
als Wachs ein natürliches Wachs verwendet wird.

8. Antimikrobielle und antioxidative sowie mikrobizide Zusammensetzung nach Anspruch 1 bis 7,
**dadurch gekennzeichnet, dass**
als Wachs ein Apfelschalenwachs, ein Bienenwachs und / oder ein Carnaubawachs sowie auch Wollwachs (Lanolin) verwendet wird.

9. Verwendung der antimikrobiellen und antioxidativen sowie mikrobizide Zusammensetzung gemäß Anspruch 1 und mindestens einem der Ansprüche 2 bis 8 zur Herstellung eines Anstrichmittels für Holz, Natur- oder Kunststein.

10. Verwendung der antimikrobiellen und antioxidativen sowie mikrobizide Zusammensetzung gemäß Anspruch 1 und mindestens einem der Ansprüche 2 bis 8 zur Herstellung eines Beschichtungs- und Imprägniermittels für Oberflächen von Textilien.

11. Verwendung der antimikrobiellen und antioxidativen sowie mikrobizide Zusammensetzung gemäß Anspruch 1 und mindestens einem der Ansprüche 2 bis 8 zur Herstellung eines Konservierungsmittels.

12. Verwendung der antimikrobiellen und antioxidativen sowie mikrobizide Zusammensetzung gemäß Anspruch 1 und mindestens einem der Ansprüche 2 bis 8 als Konservierungs- und Überzugsmittel im Lebensmittel- und Kosmetikbereich
